# EUROPEAN PATENT SPECIFICATION

(11) **EP 2 478 869 B1**
(45) Date of publication and mention of the grant of the patent: **03.05.2017**
(21) Application number: 11275138.3
(22) Date of filing: 04.11.2011
(51) Int. Cl.: A61F 2/24

(54) **Aortic valve prosthesis**
Aortenklappenprothese
Prothèse valvulaire cardiaque

(30) Priority: 05.11.2010 US 410549 P
(43) Date of publication of application: 25.07.2012
(73) Proprietor: Cook Medical Technologies LLC, Bloomington, IN 47404 (US)
(72) Inventor: Chuter, Timothy A.M., San Francisco, CA 94114 (US); Gopalakrishnamurthy, Sharath, Malleshwaram, 56003 Bangalore (IN); Roeder, Blayne A., Lafayette, IN 47909 (US); Leewood, Alan R., Lafayette, IN 47905 (US)
(74) Representative: Jehan, Robert

(56) References cited:
- WO-A1-00/41652
- WO-A1-2010/008549
- US-A1- 2002 055 772
- US-A1- 2004 055 606
- US-A1- 2007 118 209
- US-B1- 6 254 642

## Description

### Technical Field

The present invention relates to implantable medical devices, and more particularly to an implantable medical device for the repair of a damaged endoluminal valve, such as an aortic valve.

### Background of the Invention

The aortic valve functions as a one-way valve between the heart and the rest of the body. Blood is pumped from the left ventricle of the heart, through the aortic valve, and into the aorta, which in turn supplies blood to the body. Between heart contractions the aortic valve closes, preventing blood from flowing backwards into the heart.

Damage to the aortic valve can occur from a congenital defect, the natural aging process, and from infection or scarring. Over time, calcium may build up around the aortic valve causing the valve not to open and close properly. Certain types of damage may cause the valve to "leak," resulting in "aortic insufficiency" or "aortic regurgitation." Aortic regurgitation causes extra workload for the heart, and can ultimately result in weakening of the heart muscle and eventual heart failure.

After the aortic valve becomes sufficiently damaged, the valve may need to be replaced to prevent heart failure and death. One current approach involves the use of a balloon-expandable stent to place an artificial valve at the site of the defective aortic valve. Another current approach involves the positioning of an artificial valve at the site of the aortic valve using a self-expanding stent. The normal aortic valve functions well because it is suspended from above through its attachment to the walls of the coronary sinus in between the coronary orifices, and it has leaflets of the perfect size and shape to fill the space in the annulus. However, these features may be difficult to replicate with an artificial valve. The size of the implantation site depends on the unpredictable effects of the balloon dilation of a heavily calcified native valve and its annulus. Poor valve function with a persistent gradient or regurgitation through the valve may result. In addition, different radial force considerations may be needed at the different locations for the prosthesis to optimally interact with a patient's anatomy. Still further, it is important to reduce or prevent in-folding or "prolapse" of an artificial valve after implantation, particularly during diastolic pressures.

A prior art valve with reinforcement on the leaflets is known from WO 00/41652. These reinforcements help to bias the valve closed.

### Disclosure of the Invention

The present invention seeks to provide an improved valve, such as an aortic valve, and an improved medical prosthesis including a valve.

According to a first aspect of the present invention, there is provided a valve for implantation in a vessel lumen of a patient as specified in claim 1.

The reinforcement strip may have at least one material characteristic that is different relative to material characteristic of the proximal and distal regions.

The disclosed embodiments provide a valve for implantation in a patient, for example, an aortic valve. The valve comprises a proximal region comprising a cylindrical shape, and a distal region having a generally rectangular shape comprising opposing flat surfaces that are separated by narrower flat sides. A tapered region may be disposed between the proximal and distal regions, where the tapered region comprises two opposing flat surfaces that transition into the opposing flat surfaces of the distal region. The opposing flat surfaces of the tapered region are angled relative to the proximal and distal regions. The opposing flat surfaces at the distal end of the valve allow fluid flow therethrough during antegrade flow and are generally adjacent to one another to inhibit blood flow through the valve during retrograde flow.

The reinforcement strip may comprises a rectangular-shaped strip that extends along the entire tapered portion and along at least a portion of the proximal and distal regions of the valve, or alternatively may comprise diamond, elliptical or other shapes disposed at different locations of the valve.

According to a second aspect of the present invention, there is provided a prosthetic implant as specified in claim 8.

Other systems, methods, features and advantages of the invention will be, or will become, apparent to one with skill in the art upon examination of the following figures and detailed description. It is intended that all such additional systems, methods, features and advantages be within the scope of the invention, and be encompassed by the following claims.

### Brief Description of the Drawings

Embodiments of the present invention are described below, by way of example only, with reference to the accompanying drawings, in which:
FIGS. 1-2 are, respectively, side and perspective views of an exemplary stent structure in an expanded state;
FIG. 3 is a side view illustrating the stent structure of FIGS. 1-2 in a compressed state;
FIG. 4 is a side view of an exemplary integral barb of the stent structure of FIGS. 1-2;
FIGS. 5-6 are perspective views of an exemplary aortic valve when no forces are imposed upon the valve;
FIG. 7 is a perspective view the aortic valve of FIGS. 5-6 during systole;
FIGS. 8-9 are side views illustrating a technique for coupling the aortic valve of FIGS. 5-7 to the stent structure of FIGS. 1-3;
FIG. 10 is a schematic showing the aortic prosthesis of FIG. 9 disposed within a patient's anatomy;
FIGS. 11-12 are, respectively, perspective views of an aortic valve comprising suspension ties when no forces are imposed and during diastole;
FIG. 13 is a side view illustrating coupling of the aortic valve of FIGS. 11-12 to the stent structure of FIGS. 1-3;
FIGS. 14-16 are, respectively, perspective views illustrating an aortic valve comprising reinforcement strips when no forces are imposed, during systole and during diastole; and
FIGS. 17-19 illustrate aortic valves comprising one or more reinforcement strips according to another embodiment.

It is to be understood that the components shown in the drawings are not necessarily to scale, emphasis instead being placed upon illustrating the features and elements taught herein.

### Description of the Preferred Embodiments

In the present application, the term "proximal" refers to a direction that is generally closest to the heart during a medical procedure, while the term "distal" refers to a direction that is furthest from the heart during a medical procedure.

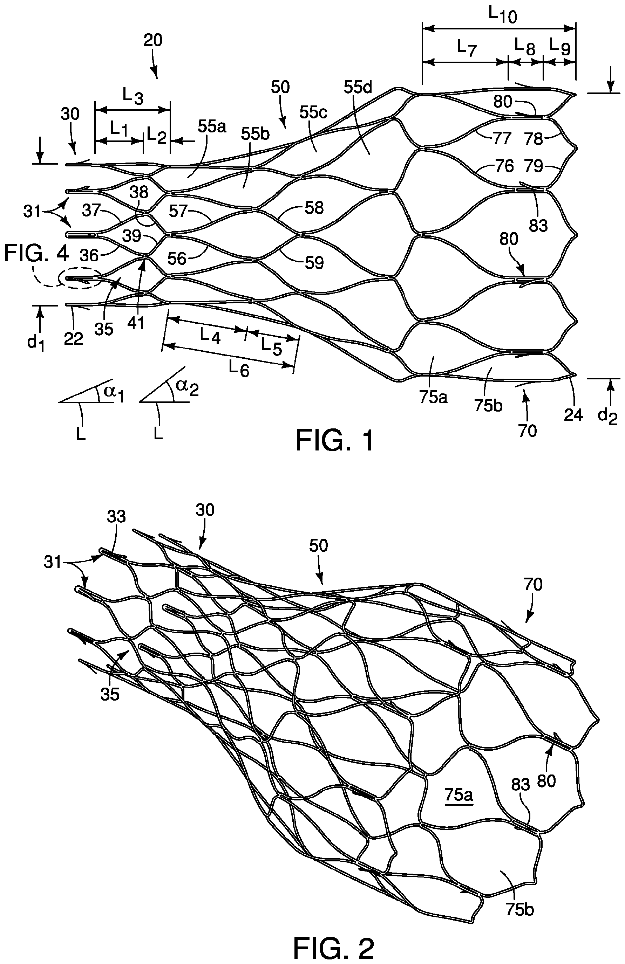

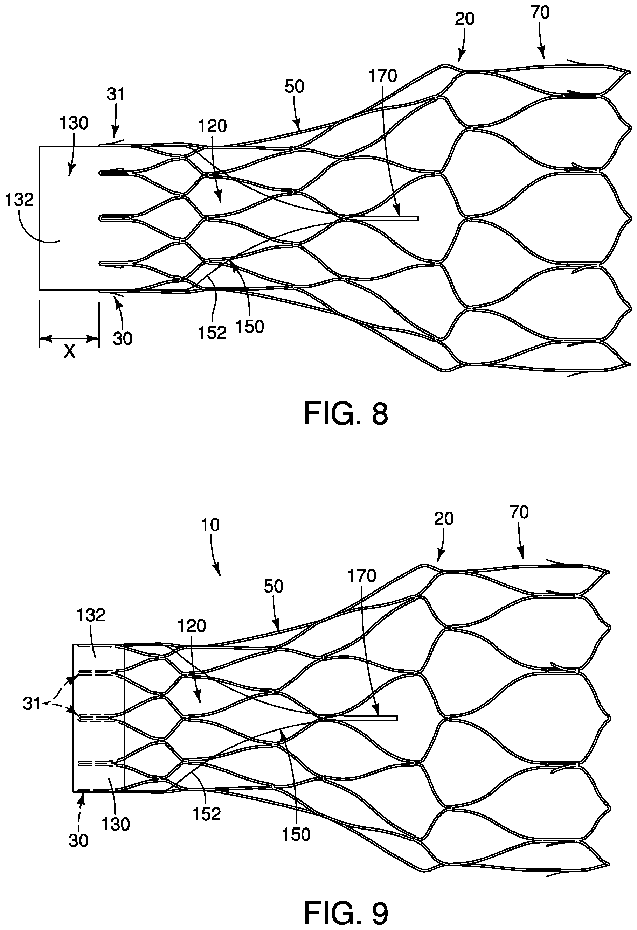

Referring now to FIGS. 1-2, a first embodiment of a stent structure 20, which may be used in conjunction with an aortic valve prosthesis, is shown and described. The stent structure 20 may be used in conjunction with an aortic valve 120 to form a completed aortic valve prosthesis 10 as shown in FIG. 9 below.

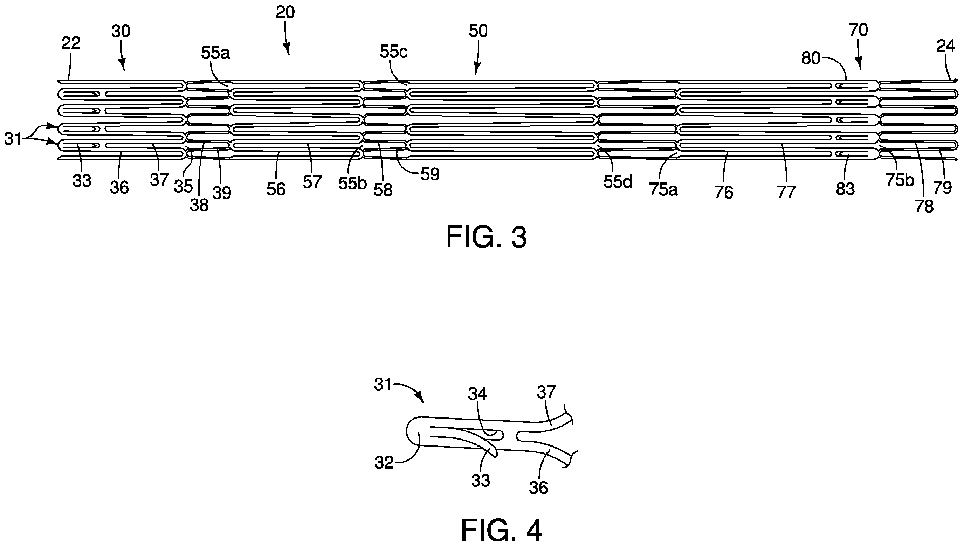

The stent structure 20 has a collapsed delivery state and an expanded deployed state, and generally comprises a proximal region 30, a tapered region 50, and a distal region 70, as shown in FIGS. 1-2. A pattern of the stent structure 20, depicted in a flattened and collapsed state, is shown in FIG. 3.

The stent structure 20 may be manufactured from a continuous cylinder into which a pattern may be cut by a laser or by chemical etching to produce slits in the wall of the cylinder. The resulting structure may then be heat set to give it a desired final configuration. As shown in FIGS. 1-2, the final configuration may include a shape having a series of multiple closed cells.

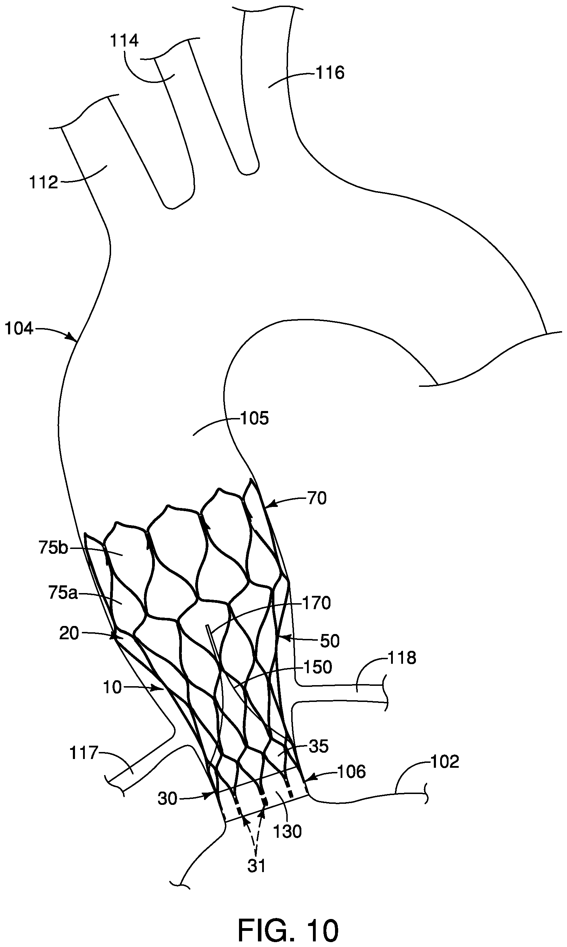

The proximal region 30 of the stent structure 20 comprises a generally cylindrical shape having an expanded outer diameter d1. The proximal region 30 is configured to be disposed at least partially within the aortic sinus, as shown in FIG. 10 below. By contrast, the distal region 70 of the stent structure 20 comprises a generally cylindrical shape having an expanded outer diameter d2, and is configured to be disposed at least partially within the ascending aorta. The tapered region 50 generally bridges the change from diameter d1 to diameter d2.

The proximal region 30 of the stent structure 20 may comprise multiple adjacent proximal apices 31. Each proximal apex 31 may comprise an end region 32 having an integral barb 33 formed therein, as shown in FIG. 4. The barb 33 may be formed by laser cutting a desired barb shape into the end regions 32. A slit 34 therefore is formed into each end region 32 after the desired barb shape is formed, as shown in FIG. 4. Once the desired barb shape is cut, a main body of the barb 33 may be bent in a radially outward direction with respect to the end region 32. The angle may comprise any acute angle, or alternatively may be substantially orthogonal or obtuse. If desired, the barb 33 may be sharpened, for example, by grinding the tip of the barb, to facilitate engagement at a target tissue site.

Referring still to FIGS. 1-2, the proximal region 30 of the stent structure 20 further may comprise a plurality of closed cells 35 formed by multiple angled strut segments. In one example, four angled strut segments 36, 37, 38 and 39 form one closed cell 35, as shown in FIG. 1. In this example, a first proximal apex 31 extends distally and splits into first and second angled strut segments 36 and 37, respectively, which are joined to one another at a junction 41. Further, third and fourth angled strut segments 38 and 39 are joined at the junction 41 and extend distally therefrom, as shown in FIG. 1. In a compressed state, the angled strut segments 36-39 of the cell 35 may be compressed such that they are substantially parallel to one another.

The first and second angled strut segments 36 and 37 each generally comprise a length L1, and each are generally disposed at an angle α1 relative to a longitudinal axis L of the stent structure 20, as shown in FIG. 1. The third and fourth angled strut segments 38 and 39 each generally comprise a length L2 and each are generally disposed at an angle α2 relative to the longitudinal axis L, as shown in FIG. 1. The closed cell 35 comprises a total length L3, representing the combined lengths L1 and L2, as shown in FIG. 1.

In this example, the length L1 of the first and second angled strut segments 36 and 37 is greater than the length L2 of the third and fourth angled strut segments 38 and 39. In one embodiment, the length L1 may be about 1.5 to about 4.0 times greater than the length L2.

Moreover, a cross-sectional area of the first and second angled strut segments 36 and 37 may be greater than a cross-sectional area of the third and fourth angled strut segments 38 and 39. In one embodiment, the cross-sectional area of the first and second angled strut segments 36 and 37 is about 4 times greater than the cross-sectional area of the third and fourth angled strut segments 38 and 39. The increased cross-sectional area of the first and second angled strut segments 36 and 37 causes these segments to primarily provide the radial force within the closed cells 35, while the third and fourth angled strut segments 38 and 39 are mainly intended for connecting adjacent closed cells 35 and 55a, instead of providing significant radial force.

Further, in this example, the angle α2 of the third and fourth angled strut segments 38 and 39 is greater than the angle α1 of the first and second angled strut segments 36 and 37. Since the first and second angled strut segments 36 and 37 are primarily providing the radial force, the angle α1 is selected to achieve the desired radial force, while as noted above, the third and fourth angled strut segments 38 and 39 are mainly intended for connecting adjacent closed cells 35 and 55a, and therefore yield a different angle α2 for this different primary purpose. In one embodiment, the angle α2 may be about 1.2 to 4.0 times greater than the angle α1.

Overall, given the relative lengths and angle configurations described above, each closed cell 35 comprises a generally spade-shaped configuration, as shown in FIGS. 1-2. As will be apparent, however, the relative lengths and angles may be greater or less than depicted and/or provided in the exemplary dimensions disclosed herein.

The pattern of angled strut segments 36-39 may be repeated around the circumference of the proximal region 30 of the stent structure 20. In this manner, the stent structure 20 may be formed into a continuous, generally cylindrical shape. In one example, ten proximal apices 31 and ten closed cells 35 are disposed around the circumference of the proximal region 30, although greater or fewer proximal apices and closed cells may be provided to vary the diameter and/or radial force characteristics of the stent.

The proximal region 30 may be flared slightly relative to the longitudinal axis L. In one example, a proximal end of each apex 31 may be bowed outward relative to a distal end of the same apex 31. Such a flaring may facilitate engagement with the aortic sinus when implanted.

Referring still to FIGS. 1-2, the tapered region 50 also comprises a plurality of closed cells. In this example, four different closed cells 55a, 55b, 55c and 55d are provided along the length of the tapered region 50. Each of the closed cells 55a-55d may comprise a slightly different shape, as shown in FIGS. 1-2. In this example, ten of each series of closed cells 55a-55d are disposed around the circumference of the tapered region 50, and the diameter of the tapered region 50 increases from the outer diameter d1 to the outer diameter d2.

In one example, four angled strut segments 56, 57, 58 and 59 form one closed cell 55b, as shown in FIG. 1. The first and second angled strut segments 56 and 57 each generally comprise a length L4 and each are generally disposed at an angle relative to the longitudinal axis L that may be about the same as, or slightly greater or less than, the angle α1. The third and fourth angled strut segments 58 and 59 each generally comprise a length L5 and each are generally disposed at an angle relative to the longitudinal axis L that may be about the same as, or slightly greater or less than, the angle α2. The closed cell 55b comprises a total length L6, representing the combined lengths L4 and L5, as shown in FIG. 1.

In this example, the length L4 of the first and second angled strut segments 56 and 57 is greater than the length L5 of the third and fourth angled strut segments 58 and 59. In one embodiment, the length L4 may be about 1.1 to about 4 times greater than the length L5.

Further, in this example, the total length L6 of the closed cell 55b of the tapered region 50 is greater than the total length L3 of the closed cell 35 of the proximal region 30, as shown in FIG. 1. Moreover, in one example, the length of one or more individual struts of the tapered region 50, e.g., first and second angled strut segments 56 and 57 having length L4, may be longer than the total length L3 of the closed cell 35 of the proximal region 30.

The distal region 70 similarly comprises a plurality of closed cells. In this example, two different closed cells 75a and 75b are provided along the length of the distal region 70. The closed cells 75a and 75b may comprise a different shape relative to one another, as shown in FIGS. 1-2. In this example, ten of each series of closed cells 75a and 75b are disposed around the circumference of the distal region 70 to form the overall outer diameter d2.

In one example, the most distal closed cell 75b comprises four angled strut segments 76, 77, 78 and 79, as shown in FIG. 1. The first and second angled strut segments 76 and 77 each generally comprise a length L7 and each are generally disposed at an angle relative to the longitudinal axis L that may be about the same as, or slightly greater or less than, the angle α1. The third and fourth angled strut segments 78 and 79 each generally comprise a length L9 and each are generally disposed at an angle relative to the longitudinal axis L that may be about the same as, or slightly greater or less than, the angle α2.

Further, a barbed region 80 having a barb 83 is disposed between the angled strut segments, as shown in FIG. 1. The barb 83 of the barbed region 80 may be formed integrally in the same manner as the barb 33 of the proximal region 30, as shown in FIG. 4, but preferably faces in a proximal direction. The barbed region 80 is generally parallel to the longitudinal axis L of the stent structure 20 and comprises a length L8. The cell 55b comprises a total length L10, representing the combined lengths L7, L8 and L9, as shown in FIG. 1.

In this example, the length L7 of the first and second angled strut segments 76 and 77 is greater than the length L9 of the third and fourth angled strut segments 78 and 79. In one embodiment, the length L7 may be about 1.1 to about 4.0 times greater than the length L9.

Further, in this example, the total length L10 of the closed cell 75b of the distal region 70 is greater than the total length L6 of the closed cell 55b of the tapered region 50, which in turn is greater than the total length L3 of the closed cell 35 of the proximal region 30, as shown in FIG. 1. Therefore, the lengths of individual closed cells increase along the stent structure from a proximal end 22 to a distal end 24 of the stent structure 20.

Advantageously, since the lengths of individual closed cells generally increase along the stent structure 20 from the proximal end 22 to the distal end 24, the forces imposed by the stent structure 20 along different regions may be varied for a patient's anatomy. Radial force and stiffness are a function of the individual cell lengths. Therefore, in the example of an aortic valve replacement, a relatively short length L3 of the closed cell 35 of the proximal region 30 yields a relatively high radial force imposed upon the aortic sinus to allow for an enhanced and rigid attachment at this location. Conversely, a relatively long length L10 of the closed cell 75b of the distal region 70 yields a relatively low radial force imposed upon the ascending aorta, thereby facilitating a flexible contour at the distal region 70 that does not adversely impact the ascending aorta 105.

Additionally, radial force and stiffness are a function of the strut angles. In the example of FIGS. 1-2, the individual struts 36 and 37 of the proximal region 30 may have a shallower strut angle relative to the individual struts 76 and 77 of the distal region 70, i.e., the individual struts 36 and 37 may be more perpendicular to the longitudinal axis L of the device. Therefore, the angles of the individual struts 36 and 37 may contribute to a higher radial force at the proximal region 30 relative to the individual struts 76 and 77 of the distal region 70.

Further, an increased strut width may be provided at the proximal region 30 to promote a higher radial force relative to the strut width at the distal region 70. In sum, the stent structure 20 has different radial force properties at its proximal and distal regions 30 and 70 that beneficially interact with their associated regions into which they are implanted, e.g., the aortic sinus and the ascending aorta, respectively.

In one embodiment, the lengths of individual cells may always increase relative to one another moving in a proximal to distal direction, i.e., each closed cell has an overall length that is greater than a length of every other closed cell that is disposed proximally thereof. In other embodiments, adjacent cells may comprise about the same length, or a proximal cell may comprise a lesser length than an adjacent distal cell. Therefore, while the lengths of individual angled strut segments generally increase in a proximal to distal direction, it is possible that some of the individual angled strut segments of a more distal region may be smaller than a more proximally oriented region.

Expansion of the stent structure 20 is at least partly provided by the angled strut segments, which may be substantially parallel to one another in a compressed state of FIG. 3, but may tend to bow outward away from one another in the expanded state shown in FIGS. 1-2. The stent structure 20 may be formed from any suitable material, and formed from a laser-cut cannula. The stent structure 20 has a reduced diameter delivery state so that it may be advanced to a target location within a vessel or duct. Further, the struts of the stent may comprise a substantially flat wire profile or may comprise a rounded profile. As best seen in FIGS. 1-2, the struts of the stent generally comprise a flat wire profile in this example.

The stent structure 20 may be manufactured from a super-elastic material. Solely by way of example, the super-elastic material may comprise a shape-memory alloy, such as a nickel titanium alloy (Nitinol). If the stent structure 20 comprises a self-expanding material such as Nitinol, the stent may be heat-set into the desired expanded state, whereby the stent structure 20 can assume a relaxed configuration in which it assumes the preconfigured first expanded inner diameter upon application of a certain cold or hot medium. The stent structure 20 may be made from other metals and alloys that allow the stent structure 20 to return to its original, expanded configuration upon deployment, without inducing a permanent strain on the material due to compression. Solely by way of example, the stent structure 20 may comprise other materials such as stainless steel, cobalt-chrome alloys, amorphous metals, tantalum, platinum, gold and titanium. The stent structure 20 also may be made from non-metallic materials, such as thermoplastics and other polymers.

It is noted that some foreshortening of the stent structure 20 may occur during expansion of the stent from the collapsed configuration of FIG. 3 to the expanded deployed state of FIGS. 1-2. Since the proximal region 30 of the stent structure 20 is deployed first, it is expected that such foreshortening is not problematic since a precise landing area of the distal region 70 within the ascending aorta is generally not needed, so long as solid contact is achieved.

Moreover, in order to reduce migration of the stent structure when implanted at a target site, it is preferred that the barbs 33 of the proximal region 30 are oriented in a distally-facing direction, whereas the barbs 83 of the distal region 70 are oriented in a proximally-facing direction. However, additional or fewer barbs may be disposed at various locations along the stent structure 20 and may be oriented in the same or different directions. Moreover, integral and/or externally attached barbs may be used.

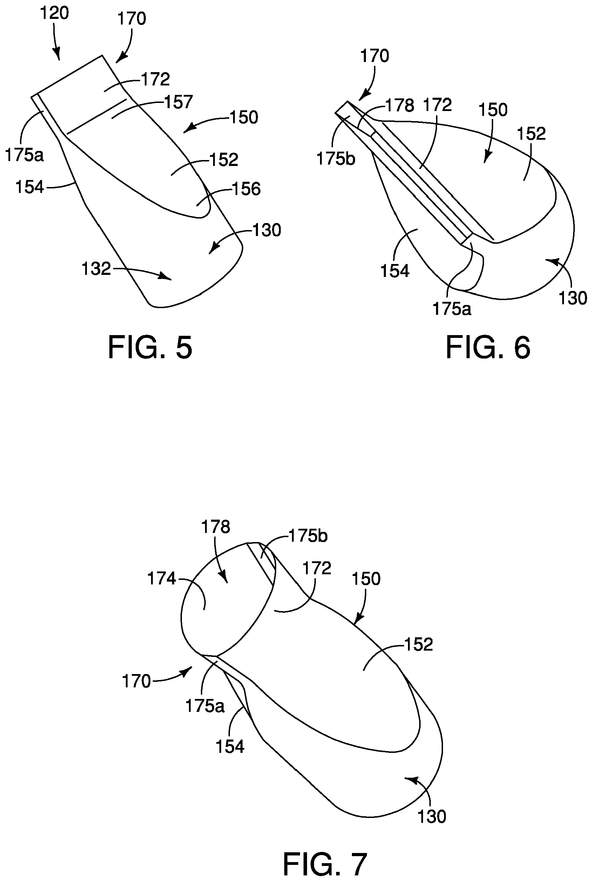

Referring now to FIGS. 5-7, a first embodiment of an aortic valve 120, which may be used in conjunction with the stent structure 20 to form an aortic prosthesis, is shown and described. The aortic valve 120 generally comprises proximal and distal regions 130 and 170, respectively, and a tapered region 150 disposed therebetween. The aortic valve 120 comprises a delivery state in which it may be compressed for percutaneous implantation along with the stent structure 20, and further comprises different states during systole and diastole. Generally, antegrade flow opens the aortic valve 120 while retrograde flow closes the aortic valve 120. In the phase of systole for the aortic valve 120, depicted in FIG. 7, blood may flow through the opposing flat surfaces 172 and 174 at the distal end 170 of the aortic valve 120. In the phase of diastole for the aortic valve 120, opposing flat surfaces 172 and 174 at the distal end 170 of the aortic valve 120 are generally adjacent to one another to inhibit blood flow back through the valve.

The proximal region 130 generally comprises a cylindrical body having an outer diameter that is approximately equal to, or just less than, an expanded inner diameter of the proximal region 30 of the stent structure 20. In one method of manufacture, shown in FIGS. 8-9 and described below, the aortic valve 120 is disposed generally within the stent structure 20 such that the proximal region 130 is at least partially aligned with the proximal region 30 of the stent structure 20.

The tapered region 150 of the aortic valve 120 may comprise two opposing flat surfaces 152 and 154, as shown in FIGS. 5-6. The opposing flat surfaces 152 and 154 generally each comprise a proximal portion 156 in the form of a curved area that reduces the diameter of the proximal region 130, and a distal portion 157 in the form of a wide flat panel that transitions into the distal region 170, as shown in FIGS. 5-6.

The distal region 170 of the aortic valve 120 may comprise a generally rectangular profile from an end view, that is looking at the device from a distal to proximal direction. The distal region 170 comprises the opposing flat surfaces 172 and 174 noted above, which are separated by narrower flat sides 175a and 175b, as shown in FIGS. 5-6. The opposing flat surfaces 152 and 154 of the tapered region 150 generally transition into the opposing flat surfaces 172 and 174 of the distal region 170, respectively. The opposing flat surfaces 152 and 154 of the tapered region 150 are angled relative to both the proximal region 130 and the distal region 170, as shown in FIGS. 5-6.

The aortic valve 120 may comprise a biocompatible graft material is preferably non-porous so that it does not leak under physiologic forces. The graft material may be formed of Thoralon® (Thoratec® Corporation, Pleasanton, California), Dacron® (VASCUTEK® Ltd., Renfrewshire, Scotland, UK), a composite thereof, or another suitable material. Preferably, the graft material is formed without seams. The tubular graft can be made of any other at least substantially biocompatible material including such fabrics as other polyester fabrics, polytetrafluoroethylene (PTFE), expanded PTFE, and other synthetic materials. Naturally occurring biomaterials are also highly desirable, particularly a derived collagen material known as extracellular matrix. An element of elasticity may be incorporated as a property of the fabric or by subsequent treatments such as crimping.

Referring to FIGS. 8-9, in one method of manufacture, the aortic valve 120 is disposed generally within the stent structure 20 such that the proximal region 130 of the aortic valve 120 is at least partially aligned with the proximal region 30 of the stent structure 20. A proximal attachment portion 132 of the aortic valve 120 having a length x is disposed proximal to the proximal apices 31 of the stent structure 20, as shown in FIG. 8, then the proximal attachment portion 132 is folded externally over the proximal apices 31, as shown in FIG. 9. The proximal attachment portion 132 then may be sutured or otherwise attached to the proximal apices 31 and/or any of the angled strut segments 36-39, thereby securing a portion of the aortic valve 120 to the stent structure 20 to form a complete aortic prosthesis 10, as depicted in FIG. 9. The barbs 33 of the stent structure 20 may protrude through the fabric of the proximal attachment portion 132 for engagement with targeted tissue.

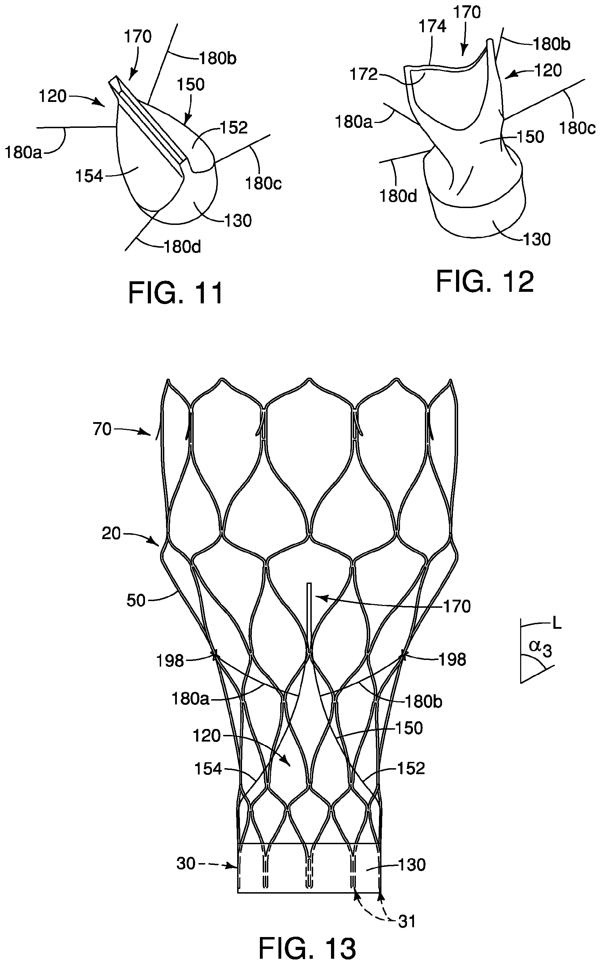

When the aortic valve 120 is coupled to the stent structure 20 as shown in FIGS. 8-9, the distal region 170 of the aortic valve 120 may extend within the tapered region 50 and/or the distal region 70 of the stent structure 20, and may be generally centrally disposed therein, although the exact positioning of distal region 170 of the aortic valve 120 relative to the stent structure 20 may be varied as needed. Moreover, one or more reinforcement members, described generally in FIGS. 11-19 below, may be coupled to the aortic valve 120 and/or the stent structure 20 to enhance structural integrity and/or functionality of the aortic prosthesis 10.

Advantageously, the distal region 170 of the aortic valve 120 is disposed within the tapered region 50 and/or the distal region 70 of the stent structure 20, which are positioned in the proximal ascending thoracic aorta above (distal to) the annulus and above the native aortic valve. Previous valves are designed to occupy the aortic annulus; however, the unpredictable shape and diameter of the aortic annulus makes the valve unpredictable in shape and diameter, leading to asymmetric replacement valve movement, leakage and reduced durability. In short, by moving the distal region 170 of the aortic valve 120 to a distally spaced-apart location relative to the native aortic valve, the unpredictable shape and diameter of the aortic annulus have less impact upon the spaced-apart distal region 170 of the aortic valve 120, and therefore the distal region 170 is less subject to asymmetric valve movement and leakage, and may have increased durability.

The shape and dimensions of the proximal and tapered regions 130 and 150 can vary without significantly affecting flow or valve function at the distal region 170. While the distal region 170 of the valve 120 is shown having a generally rectangular shape, a tricuspid-shaped distal region of the valve may be provided, in which case the tapered region 150 may be omitted or altered to accommodate such a tricuspid-shaped distal region.

Referring now to FIG. 10, a partial cut-away view of a heart 102 and an aorta 104 are shown. The heart 102 may comprise an aortic valve 106 that does not seal properly. This defect of the aortic valve 106 allows blood to flow from the aorta 104 back into the left ventricle, leading to a disorder known as aortic regurgitation. Also shown in FIG. 10 are a brachiocephalic trunk 112, a left common carotid artery 114, and a left subclavian artery 116. A portion of the aorta 104 referred to herein as an ascending aorta 105 is shown located between the aortic valve 106 and the brachiocephalic trunk 112. A patient's coronary arteries 117 and 118 are located distal to the aortic valve 106.

The aortic prosthesis 10 is introduced into a patient's vascular system, delivered, and deployed using a deployment device, or introducer. The deployment device delivers and deploys the aortic prosthesis 10 within the aorta at a location to replace the aortic valve 106, as shown in FIG. 10. The deployment device may be configured and sized for endoluminal delivery and deployment through a femoral cut-down. The aortic prosthesis 10, with the stent structure 20 in a radially collapsed state, may be inserted into a delivery catheter using conventional methods. In addition to a delivery catheter, various other components may need to be provided in order to obtain a delivery and deployment system that is optimally suited for its intended purpose. These include and are not limited to various outer sheaths, pushers, trigger wires, stoppers, wire guides, and the like. For example, the Zenith® Thoracic Aortic Aneurysm Endovascular Graft uses a delivery system that is commercially available from Cook Inc., Bloomington, Indiana, and may be suitable for delivering and deploying an aortic prosthesis in accordance with the present embodiments.

In one embodiment, a trigger wire release mechanism is provided for releasing a retained end of the stent structure 20 of the aortic prosthesis 10. Preferably, the trigger wire arrangement includes at least one trigger wire extending from a release mechanism through the deployment device, and the trigger wire is engaged with selected locations of the stent structure 20. Individual control of the deployment of various regions of the stent structure 20 enables better control of the deployment of the aortic prosthesis 10 as a whole.

While the stent structure 20 is generally described as a self-expanding framework herein, it will be appreciated that a balloon-expandable framework may be employed to accomplish the same functionality. If a balloon-expandable stent structure is employed, then a suitable balloon catheter is employed to deliver the aortic prosthesis as generally outlined above. Optionally, after deployment of a self-expanding stent structure 20, a relatively short balloon expandable stent may be delivered and deployed inside of the proximal region 30 of the stent structure 20 to provided added fixation at the location of the aortic sinus.

Upon deployment, the aortic prosthesis 10 is positioned as generally shown in FIG. 10. Advantageously, as noted above, since the lengths of individual cells generally increase along the stent structure 20 from the proximal end 22 to the distal end 24, a relatively high radial force is imposed by the closed cells 35 of the proximal region 30 upon the aortic sinus 106 to allow for an enhanced and rigid attachment at this location. Conversely, a relatively low radial force is imposed by the closed cells 75a and 75b of the distal region 70 upon the ascending aorta 105, thereby facilitating a flexible contour at the distal region that does not adversely impact the ascending aorta 105.

When the aortic prosthesis 10 is implanted, sufficient flow into the coronary arteries 117 and 118 is maintained during retrograde flow. In particular, after blood flows through the distal region 170 of the aortic valve 120, blood is allowed to flow adjacent to the outside of the tapered central region 150 of the aortic valve 120 and into the coronary arteries 117 and 118, i.e., through the open individual cells of the stent structure 20.

Further, if the barbs 33 are disposed at the proximal region 30, the barbs 33 promote a secure engagement with the aortic sinus 106. Similarly, the barbs 83 at the distal region 70 promote a secure engagement with the ascending aorta 105. In the event barbs are omitted, the proximal and distal regions 30 and 70 may be configured so that the radial forces exerted upon the coronary sinus 105 and the ascending aorta 105, respectively, are enough to hold the stent structure 20 in place.

The shape, size, and dimensions of each of the members of the aortic prosthesis 10 may vary. The size of a preferred prosthetic device is determined primarily by the diameter of the vessel lumen (preferably for a healthy valve/lumen combination) at the intended implant site, as well as the desired length of the overall stent and valve device. Thus, an initial assessment of the location of the natural aortic valve in the patient is determinative of several aspects of the prosthetic design. For example, the location of the natural aortic valve in the patient will determine the dimensions of the stent structure 20 and the aortic valve 120, the type of valve material selected, and the size of deployment vehicle.

After implantation, the aortic valve 120 replaces the function of the recipient's native damaged or poorly performing aortic valve. The aortic valve 120 allows blood flow when the pressure on the proximal side of the aortic valve 120 is greater than pressure on the distal side of the valve. Thus, the artificial valve 120 regulates the unidirectional flow of fluid from the heart into the aorta.

Referring now to FIGS. 11-19, various reinforcement members are described that may be coupled to the aortic valve 120 and/or the stent structure 20 to enhance structural integrity and/or functionality of the aortic prosthesis 10. The normal, native aortic valve is suspended from above through its attachment to the walls of the coronary sinus, and suspended aortic valves resist the forces created by diastolic pressure on closed leaflets through attachment to downstream support. The various reinforcement members of FIGS. 11-19 are intended to reinforce the aortic valve 120, and in particular, prevent in-folding or "prolapse" of the valve during diastole.

In FIGS. 11-13, a first embodiment of reinforcement members comprises a plurality of suspension ties 180a-180d that are coupled between the tapered region 150 of the aortic valve 120 and the tapered region 50 of the stent structure 20. In the phase of systole for the aortic valve 120, blood may flow through the opposing flat surfaces 172 and 174 at the distal end 170 of the aortic valve 120, and the suspension ties 180a-180d are relatively slack allowing for normal opening of the aortic valve 120. In the phase of diastole for the aortic valve 120, opposing flat surfaces 172 and 174 at the distal end 170 of the aortic valve 120 are generally adjacent to one another to inhibit blood flow back through the valve, while the suspension ties 180a-180d become more taut and prevent prolapse of the aortic valve 120 when retrograde flow is imposed upon the exterior surfaces of the valve, as depicted in the finite element analysis simulation of FIG. 12. In effect, the suspension ties 180a-180d advantageously provide a safety mechanism by which prolapse is avoided during retrograde flow.

In the example of FIGS. 11-13, the suspension ties 180a-180d may be molded into the aortic valve 120 in the manner that fiber reinforcements are molded into a graft structure, and further may be coupled to one or more struts of the stent structure 20 using sutures 198 or another suitable coupling member that does not impede expansion of the stent structure 20. While first ends of the suspension ties 180a-180d are shown coupled to the tapered region 150 of the aortic valve 120, they may alternatively, or additionally, be coupled to another location, such as the distal region 170. Similarly, while second ends of the suspension ties 180a-180d are shown coupled to the tapered region 50 of the stent structure 20, they may alternatively, or additionally, be coupled to another location, such as the distal region 70. While four exemplary suspension ties 180a-180d are shown, greater or fewer suspension ties may be used, and their positioning may be varied as noted above, to achieve the desired functionality and reduce potential prolapse of the aortic valve 120.

The angles α3 of the suspension ties 180a-180d relative to the longitudinal axis L, as shown in FIG. 13, may be between about 40-80 degrees when relatively slack. However, it will be appreciated the angles α3 may be greater or less than what is depicted in FIG. 13.

In one example, the suspension ties 180a-180d comprise a thickness of between about 0.0508 - 0.508 mm (0.002 - 0.02 inches), and are molded into a Thoralon® or Dacron® coating. Other materials may be used, so long as the suspension ties 180a-180d are non-thrombogenic, or coated with a non-thrombogenic material.

Advantageously, in the case where the tapered or distal regions 150 and 170 of the aortic valve 120 are supported from above through attachment to the stent structure 20 at a location in the ascending thoracic aorta, the aortic valve 120 can therefore be as long as necessary for optimal valve function, even if it is of a simple bicuspid design. In other words, the length of the aortic valve 120 can be varied such that the distal region 170 of the aortic valve 120 is positioned at the desired location within the ascending thoracic aorta spaced-apart from the native aortic annulus.

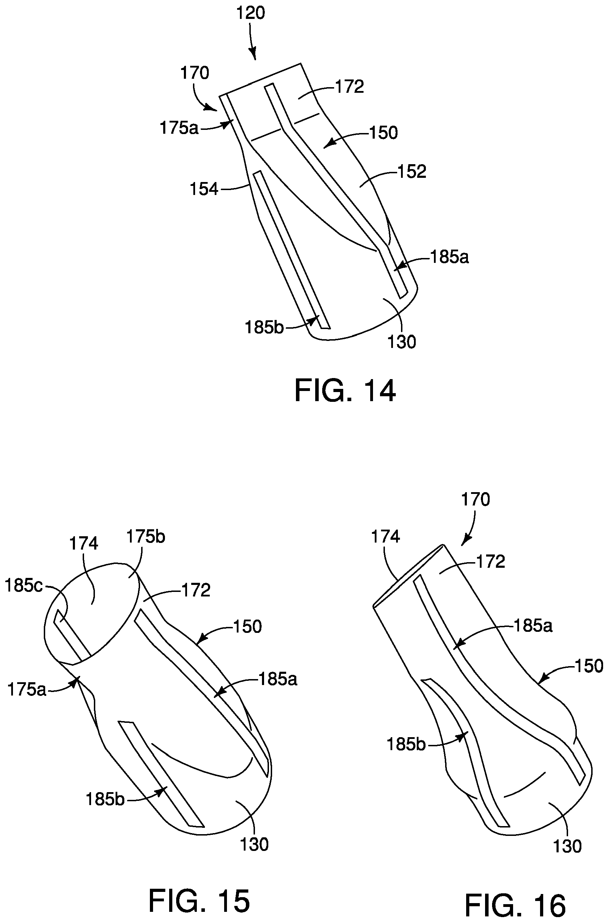

Referring now to FIGS. 14-19, other embodiments of reinforcement members are shown and described that comprise one or more reinforcement strips. In FIGS. 14-16, a first reinforcement strip 185a generally extends between a portion of the proximal region 130, through one opposing flat surface 152 of the tapered region 150, and to one opposing flat surface 172 of the distal region 170, as shown in FIGS. 14-16. A second reinforcement strip 185b is disposed about 90 degrees apart from the first reinforcement strip 185a, and generally extends between a portion of the proximal region 130 towards one of the narrower flat sides 175a. A third reinforcement strip 185c is disposed about 90 degrees apart from the first reinforcement strip 185a, and generally extends between a portion of the proximal region 130, through one opposing flat surface 154 of the tapered region 150, and to one opposing flat surface 174 of the distal region 170. A fourth reinforcement strip is obscured in FIGS. 14-16 but may be disposed about 90 degrees apart from the second reinforcement strip 185b and is a mirror image thereof.

In the phase of systole for the aortic valve 120, shown in FIG. 15, blood may flow through the opposing flat surfaces 172 and 174 at the distal end 170 of the aortic valve 120, and the reinforcement strips 185a-185c are relatively flat allowing for normal opening of the aortic valve 120. In the phase of diastole for the aortic valve 120, shown in FIG. 16, opposing flat surfaces 172 and 174 at the distal end 170 of the aortic valve 120 are generally adjacent to one another to inhibit blood flow back through the valve, while the reinforcement strips 185a-185d may become bowed radially inward along the tapered region 150 to prevent prolapse of the aortic valve 120 when retrograde flow is imposed upon the exterior surfaces of the valve. In one example, the reinforcement strips 185a-185c may snap between the states depicted in FIGS. 15-16 during systole and diastole, respectively, when the associated pressures are imposed upon the aortic valve 120. In effect, the reinforcement strips 185a-185c advantageously provide a safety mechanism by which prolapse is avoided during retrograde flow.

In one example, the reinforcement strips 185a-185c of FIGS. 14-16 comprise stainless steel or Nitinol, though any suitable material to perform such functions may be used. The reinforcement strips may comprise a thickness of about 0.0508 - 0.245 mm (0.002 to about 0.010 inches) and may be molded into the material of the aortic valve 120, or coupled externally thereto.

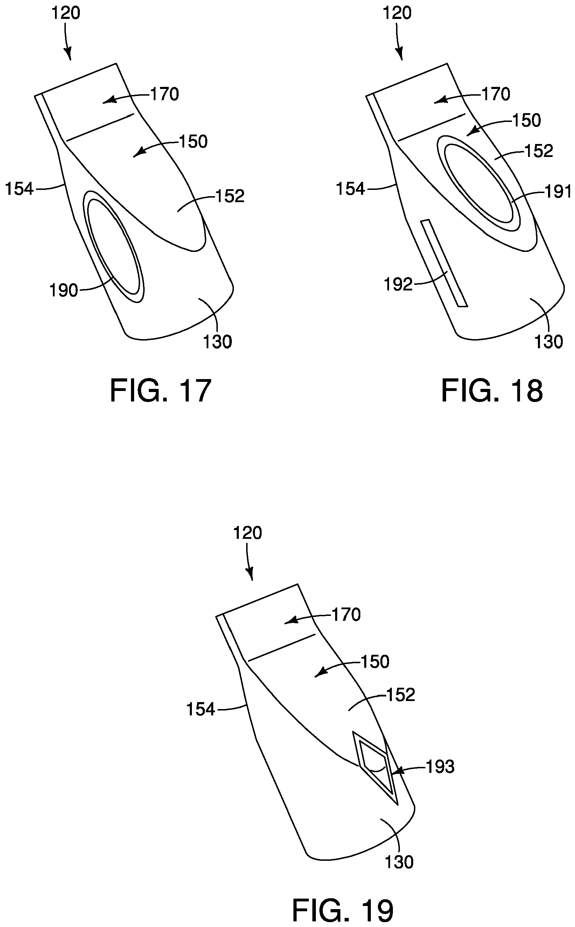

In FIGS. 17-19, various other embodiments of reinforcement strips are depicted. In FIG. 17, at least one elliptical reinforcement strip 190 is coupled to a portion of the proximal region 130 of the aortic valve 120 and extends distally into the tapered region 150, positioned generally between the opposing flat surfaces 152 and 154 of the tapered region 150. In FIG. 18, a first elliptical reinforcement strip 191 is coupled entirely to the flat surface 152 of the tapered region 150, while a second longitudinal reinforcement strip 192 extends between the proximal region 130 and tapered region 150 and is positioned generally between the opposing flat surfaces 152 and 154 of the tapered region 150. In FIG. 19, a diamond-shaped reinforcement strip 193 is coupled between the proximal region 130 and the flat surface 152 of the tapered region 150. Like the reinforcement strips 185a-185c of FIGS. 14-16, the reinforcement strips 190-193 of FIGS. 17-19 may snap between two states during systole and diastole. In each of the embodiments of FIGS. 17-19, the reinforcement strips 190-193 advantageously provide a safety mechanism by which prolapse is avoided during retrograde flow. While various exemplary reinforcement strip shapes and locations are shown in FIGS. 14-19, the shapes and locations of the reinforcement strips may be varied, and greater or fewer strips may be used.

In still further embodiments, the stent structure 20 shown herein may be used in connection with different aortic valves, beside the aortic valve 120. Solely by way of example, and without limitation, various artificial valve designs may have two or three membranes, and may be arranged in various shapes including slots and flaps that mimic the natural functionality of an anatomical valve. Conversely, the aortic valve 120 shown herein may be used in conjunction with different stent structures.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

It is also to be understood that the features of the various embodiments described herein may be combined with one another.

## Claims

1. A valve (120) for implantation in a vessel lumen of a patient, the valve including:
a proximal region (130) comprising a cylindrical body;
a distal region (170) having a generally rectangular shape comprising opposing flat surfaces (172, 174) that are separated by narrower flat sides, where the opposing flat surfaces at the distal end of the valve allow fluid flow therethrough during antegrade flow and are generally adjacent to one another to inhibit blood flow through the valve during retrograde flow, and
at least one reinforcement member comprising a reinforcement strip (185; 190; 191, 192; 193) coupled to the valve and operable to prevent prolapse of the valve during retrograde flow, wherein the reinforcement strip snaps between a first state during antegrade flow and a second state during retrograde flow.

2. A valve (120) according to claim 1, wherein the reinforcement strip (185; 190; 191, 192; 193) has at least one material characteristic that is different relative to material characteristic of the proximal and distal regions (130; 170).

3. A valve (120) according to claim 2, including a tapered region (150) disposed between the proximal and distal regions (130, 170), where the tapered region comprises opposing flat surfaces (152, 154) that transition into the opposing flat surfaces (172, 174) of the distal region, and where the opposing flat surfaces of the tapered region are angled relative to the proximal and distal regions.

4. A valve (120) according to claim 3, wherein the reinforcement strip (185; 190; 191, 192; 193) includes a rectangular-shaped strip that extends along the entire tapered portion (150) and along at least a portion of the proximal and distal regions (130, 170).

5. A valve (120) according to claim 2, 3 or 4, wherein at least one reinforcement strip (193) comprises a diamond shape and an elliptical shape.

6. A valve according to any of claims 2 to 5, wherein at least one reinforcement strip (19) comprises an elliptical shape.

7. A valve according to any of claims 2 to 6, wherein a first reinforcement strip comprises a first shape that is different than a second shape of a second reinforcement strip.

8. A prosthetic implant including a valve (120) according to any preceding claim.

## Patentansprüche

1. Klappe (120) zur Implantation in ein Gefäßlumen eines Patienten, wobei die Klappe Folgendes aufweist:
einen proximalen Bereich (130), der einen zylindrischen Körper umfasst,
einen distalen Bereich (170) mit einer allgemein rechteckigen Form, der sich gegenüberliegende flache Oberflächen (172, 174) umfasst, die durch schmalere flache Seiten getrennt sind, wobei die sich gegenüberliegenden flachen Oberflächen am distalen Ende der Klappe während der antegraden Strömung Fluidströmung dort hindurch gestatten und einander allgemein benachbart sind, um während der retrograden Strömung die Blutströmung durch die Klappe zu verhindern, und
mindestens ein Verstärkungsglied, das einen Verstärkungsstreifen (185; 190; 191, 192; 193) umfasst, der an die Klappe gekoppelt und dahingehend betätigbar ist, einen Klappenvorfall während der retrograden Strömung zu verhindern, wobei der Verstärkungsstreifen zwischen einem ersten Zustand während der antegraden Strömung und einem zweiten Zustand während der retrograden Strömung schnappt.

2. Klappe (120) nach Anspruch 1, wobei der Verstärkungsstreifen (185; 190; 191, 192; 193) mindestens eine Materialeigenschaft hat, die sich von der Materialeigenschaft des proximalen und des distalen Bereichs (130; 170) unterscheidet.

3. Klappe (120) nach Anspruch 2, die einen sich verjüngenden Bereich (150) aufweist, der zwischen dem proximalen und dem distalen Bereich (130, 170) angeordnet ist, wobei der sich verjüngende Bereich sich gegenüberliegende flache Oberflächen (152, 154) umfasst, die in die sich gegenüberliegenden flachen Oberflächen (172, 174) des distalen Bereichs übergehen, und wobei die sich gegenüberliegenden flachen Oberflächen des sich verjüngenden Bereichs bezüglich des proximalen und des distalen Bereichs abgewinkelt sind.

4. Klappe (120) nach Anspruch 3, wobei der Verstärkungsstreifen (185; 190; 191, 192; 193) einen rechteckigen Streifen aufweist, der sich entlang dem gesamten sich verjüngenden Abschnitt (150) und entlang mindestens einem Abschnitt des proximalen und des distalen Bereichs (130, 170) erstreckt.

5. Klappe (120) nach Anspruch 2, 3 oder 4, wobei mindestens ein Verstärkungsstreifen (193) eine Rautenform und eine elliptische Form umfasst.

6. Klappe nach einem der Ansprüche 2 bis 5, wobei mindestens ein Verstärkungsstreifen (19) eine elliptische Form umfasst.

7. Klappe nach einem der Ansprüche 2 bis 6, wobei ein erster Verstärkungsstreifen eine erste Form umfasst, die sich von einer zweiten Form eines zweiten Verstärkungsstreifens unterscheidet.

8. Prothesenimplantat, das eine Klappe (120) nach einem der vorhergehenden Ansprüche aufweist.

## Revendications

1. Valve (120) destinée à être implantée dans une lumière vasculaire d'un patient, la valve comprenant :
une région proximale (130) comprenant un corps cylindrique ;
une région distale (170) présentant une forme globalement rectangulaire comprenant des surfaces planes opposées (172, 174) qui sont séparées par des côtés plats plus étroits, les surfaces planes opposées au niveau de l'extrémité distale de la valve permettant un écoulement de fluide à travers elles lors d'un écoulement antérograde et sont globalement adjacentes l'une à l'autre de façon à inhiber l'écoulement de sang à travers la valve lors d'un écoulement rétrograde, et
au moins un élément de renforcement comprenant une bande de renforcement (185 ; 190 ; 191, 192 ; 193) accouplée à la valve et pouvant être mise en oeuvre pour empêcher un prolapsus de la valve lors d'un écoulement rétrograde, dans laquelle la bande de renforcement passe brusquement entre un premier état lors d'un écoulement antérograde et un second état lors d'un écoulement rétrograde.

2. Valve (120) selon la revendication 1, dans laquelle la bande de renforcement (185 ; 190 ; 191, 192 ; 193) présente au moins une caractéristique matérielle qui est différente d'une caractéristique matérielle des régions proximale et distale (130 ; 170).

3. Valve (120) selon la revendication 2, comprenant une région effilée (150) disposée entre les régions proximale et distale (130, 170), la région effilée comprenant des surfaces planes opposées (152, 154) qui deviennent les surfaces planes opposées (172, 174) de la région distale, et les surfaces planes opposées de la région effilée étant obliques par rapport aux régions proximale et distale.

4. Valve (120) selon la revendication 3, dans laquelle la bande de renforcement (185 ; 190 ; 191, 192 ; 193) comprend une bande de forme rectangulaire qui s'étend le long de l'intégralité de la partie effilée (150) et le long d'au moins une partie des régions proximale et distale (130, 170).

5. Valve (120) selon la revendication 2, 3 ou 4, dans laquelle au moins une bande de renforcement (193) comprend une forme en losange et une forme elliptique.

6. Valve selon l'une quelconque des revendications 2 à 5, dans laquelle au moins une bande de renforcement (19) comprend une forme elliptique.

7. Valve selon l'une quelconque des revendications 2 à 6, dans laquelle une première bande de renforcement comprend une première forme qui est différente d'une seconde forme d'une seconde bande de renforcement.

8. Implant prothétique comprenant une valve (120) selon l'une quelconque des revendications précédentes.
